# FASCICULE DE BREVET EUROPEEN

(11) **EP 4 452 060 B1**
(45) Date de publication et mention de la délivrance du brevet: **21.01.2026**
(21) Numéro de dépôt: 22847595.0
(22) Date de dépôt: 20.12.2022
(51) Int. Cl.: A61B 5/0536, A61B 5/00, G01N 27/02, G01R 27/02

(54) **DISPOSITIF DE MESURE DE TOMOGRAPHIE PAR PROCESSUS ÉLECTRIQUE AMELIORÉ**
VERBESSERTE ELEKTRISCHE TOMOGRAPHIEMESSVORRICHTUNG
IMPROVED ELECTRICAL TOMOGRAPHY MEASUREMENT DEVICE

(30) Priorité: 24.12.2021 FR 2114482
(43) Date de publication de la demande: 30.10.2024
(73) Titulaire: Touch Sensity, 33600 Pessac (FR)
(72) Inventeur: FREITAS, Philippe, 33702 Merignac Cedex (FR); PUGACH, Ganna, 33702 Merignac Cedex (FR); PASSET, Charles, 33702 Merignac Cedex (FR)
(74) Mandataire: Cabinet Netter
(86) Numéro de dépôt international: PCT/FR2022/052441
(87) Numéro de publication internationale: WO 2023/118732

(56) Documents cités:
- US-A1- 2009 216 148
- HUBBARD DYLAN ET AL: "ADC Voltage Reference Buffer Optimization Reference Design for High- Performance DAQ Systems ADC Voltage Reference Buffer Optimization Reference Design for High-Performance DAQ Systems", 31 January 2018 (2018-01-31), XP055944309, Retrieved from the Internet <URL:https://www.ti.com/lit/ug/tidude2a/tidude2a.pdf?ts=1658242927042&ref_url=https%3A%2F%2Fwww.google.com%2F> [retrieved on 20220719]

## Description

L'invention concerne le domaine de la tomographie par processus électrique (TPE, pour « Electrical Process Tomography » en anglais).

La tomographie par processus électrique, qui comprend la tomographie par capacité électrique (TCE), la tomographie par impédance électrique (TIE) et la tomographie par résistance électrique (TRE), est basée sur les propriétés spécifiques des matériaux principalement détectées par chaque technique :
- la TIE est utile pour un processus qui a une phase continue conductrice, et
- la tomographie par résistance électrique (TRE) est un cas particulier de la TIE, lorsque la composante réelle de l'impédance électrique est la propriété dominante des matériaux,
- la TCE détecte la distribution de la permittivité des matériaux dispersés dans un processus avec une phase continue peu conductrice.

La TPE a connu des applications à la mesure de fluides, en particulier dans le cas de processus industriels. Dans le cas de la TIE, le capteur est constitué de plusieurs électrodes disposées à la périphérie de la paroi interne de la cuve du procédé ou de la canalisation, en contact avec le fluide du procédé mais sans intrusion dans le fluide. Un courant alternatif est appliqué à certaines électrodes et des tensions sont mesurées à partir des autres électrodes, selon une stratégie de détection prédéfinie. Ensuite, ces mesures de tension sont utilisées pour reconstruire la distribution d'impédance à l'intérieur de la cuve avec un algorithme inverse spécifique.

Dans le cas de substrat à mesurer solide (c'est-à-dire non liquide et non gazeux), les développements sont plus rares. La TIE appliquée à un substrat solide est basée sur la reconstruction du champ électrique d'une partie conductrice du substrat. Cette technique non-invasive est utilisée en imagerie médicale pour détecter les corps internes en appliquant les électrodes à la surface de la peau d'un patient et en mesurant les variations du champ électrique.

Hors du champ médical, la TIE appliquée à un substrat solide a connu quelques utilisations dans le domaine de la détection de pressions. Ainsi, elle a été utilisée dans les articles de Kato et al. ("Tactile sensor without wire and sensing element in the tactile region based on eit method", IEEE Sensors, pages 792-795, 2007), et de Yao et Soleimani ("A pressure mapping imaging device based on electrical impedance tomography of conductive fabrics", Sensor Review, 32(4):310-317, 2012) pour proposer des capteurs tactiles (capteurs de pression). Les articles de Nagakubo et al. ("A deformable and deformation sensitive tactile distribution sensor", IEEE International Conference on Robotics and Biomimetics, ROBIO, pages 1301-1308, 2007), de Alirezaei et al. ("A highly stretchable tactile distribution sensor for smooth surfaced humanoids", 7th IEEE-RAS International Conference on Humanoid Robots, pages 167-173, 2007 et "A tactile distribution sensor which enables stable measurement under high and dynamic stretch", IEEE Symposium on 3D User Interfaces (3DUI), pages 87-93, 2009), et de Tawil et al. ("Improved image reconstruction for an eit-based sensitive skin with multiple internal electrodes", IEEE Transactions on Robotics, 27(3):425-435, 2011), ont proposé des dispositifs tactiles de type « peau artificielle » pour robots. Leur approche consiste à injecter des courants et à mesurer des tensions à partir d'électrodes connectées sur les bords d'un tissu conducteur, puis à appliquer l'analyse du problème inverse pour reconstruire le changement local de la résistivité due à une pression. Enfin, les articles de Pugach et al. ("Electronic hardware design of a low cost tactile sensor device for physical human-robot interactions", IEEE XXXIII International Scientific Conference Electronics and Nanotechnology, ELNANO, pages 445-449, 2013, "Neural learning of the topographic tactile sensory information of an artificial skin through a self-organizing map", Advanced Robotics, 29(21):1393-1409, 2015, et "Touch-based admittance control of a robotic arm using neural learning of an artificial skin", in 2016 IEEE/RSJ International Conference on Intelligent Robots and Systems (IROS), pages 3374-3380, 2016) ont décrit l'utilisation de réseaux de neurones pour reconstruire la distribution de résistance au sein d'un film conducteur et localiser des points de pression.

D'autres applications ont été faites dans la détection de défauts structurels dans le ciment. Ainsi, l'article de Milad Hallaji et al. « Electrical impedance tomography-based sensing skin for quantitative imaging of damage in concrete » 2014 Smart Mater. Struct. 23 085001 et la thèse de Kimmo Karhunen « Electrical resistance tomography imaging of concrete », 2013, Publications of the University of Eastern Finland Dissertations in Forestry and Natural Sciences No 122 montrent des explorations d'extensions de la TIE à des matériaux dont la mesure de conductivité est plus compliquée que celle d'un tissu ou de la peau, ainsi que les limites de telles mesures. Le document US 2009/216148 A1 (FREED ET AL, 2009-08-27) décrit les caractéristiques de la première partie de la revendication 1.

La TIE appliquée à des substrats solides nécessite donc un matériau conducteur permettant d'observer des variations d'impédance suffisamment importantes, ainsi qu'une bonne capacité à relier les électrodes pour la mesure. Dans le cas de matériaux à faible variation d'impédance ou complexes à relier à des électrodes, la TIE et la TPE en général sont proscrites, car il est trop complexe d'obtenir une résolution suffisante pour extraire une information des données de mesure.

L'invention vient améliorer la situation. À cet effet, elle propose un dispositif de mesure de tomographie par processus électrique pour substrat solide, comprenant une source de courant ou de tension, une pluralité d'électrodes propres à être reliées à un substrat solide à mesurer et un convertisseur analogique numérique comprenant deux entrées de mesure et une entrée de référence, agencé pour émettre un signal numérique correspondant à la différence entre la tension des deux entrées de mesure proportionnellement à un niveau de tension désigné par l'entrée de référence, la source de courant ou de tension pouvant être reliée à une paire d'électrodes dans la pluralité d'électrodes de manière commandée, de sorte qu'un courant issu de la source de courant ou de tension traverse ledit substrat à mesurer selon une séquence d'excitation choisie, les deux entrées de mesure du convertisseur analogique numérique pouvant chacune être reliée à une électrode respective d'une paire d'électrodes dans la pluralité d'électrodes choisie en fonction de ladite séquence d'excitation, caractérisé en ce que la source de courant ou de tension est reliée à l'entrée de référence du convertisseur analogique numérique.

Ce dispositif est particulièrement avantageux car il permet d'utiliser la TPE et plus particulièrement la TIE pour des applications de type interface homme-machine, du contrôle non destructif ou pour de la surveillance de structure, y compris avec des matériaux qui ne se prêtent normalement pas à la TPE ou la TIE.

En effet, l'utilisation du signal tiré de la source de courant ou de tension comme signal de référence pour le convertisseur analogique numérique permet d'annuler dans la mesure le bruit dû au signal de la stimulation lui-même. Cela permet d'augmenter de manière très importante la résolution des mesures et de faire usage de TPE dans des contextes inaccessibles précédemment.

La portée de l'invention est définie par les revendications annexées.

D'autres caractéristiques et avantages de l'invention apparaîtront mieux à la lecture de la description qui suit, tirée d'exemples donnés à titre illustratif et non limitatif, tirés des dessins sur lesquels :
- la Figure 1 représente un schéma générique de mise en œuvre d'un dispositif de TPE,
- la Figure 2 représente un schéma d'un premier mode de réalisation d'un dispositif de mesure selon l'invention,
- la Figure 3 représente un schéma d'un deuxième mode de réalisation d'un dispositif de mesure selon l'invention,
- la Figure 4 représente un schéma d'un troisième mode de réalisation d'un dispositif de mesure selon l'invention,
- la Figure 5 représente un schéma d'un quatrième mode de réalisation d'un dispositif de mesure selon l'invention,
- la Figure 6 représente un schéma d'un cinquième mode de réalisation d'un dispositif de mesure selon l'invention, et
- la Figure 7 représente un schéma d'un sixième mode de réalisation d'un dispositif de mesure selon l'invention.

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

L'invention concerne les mesures de la TPE et de la TIE utilisées par exemple pour la surveillance de l'état des structures SHM, le CND et les IHM. Ces systèmes visent à surveiller la variation dans le temps d'un élément physique spécifique.

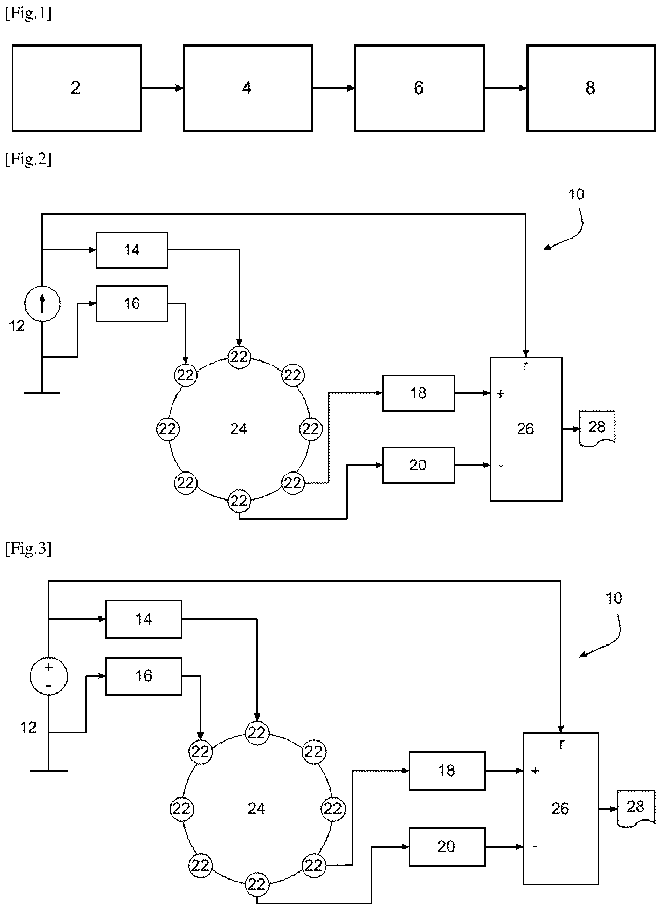

Comme représenté sur la figure 1, ces systèmes sont généralement composés d'un sous-système de stimulation 2, d'un élément ou substrat à surveiller 4, d'un sous-système de d'acquisition de données 6 intégrant un convertisseur analogique-numérique, et d'un système de traitement des données 8 pour déterminer la variation dans le temps de l'élément physique spécifique. Le système de traitement des données 8 n'est pas l'objet principal de l'invention. C'est l'ensemble permettant d'acquérir les mesures, jusqu'au sous-système d'acquisition 6 qui l'est. En variante, le sous-système de d'acquisition de données 6 peut comprendre des opérations d'amplification et de filtrage préalables à la conversion analogique-numérique.

Le sous-système de stimulation 2 a pour but de générer un signal de stimulation, celui-ci étant de nature électrique, et d'appliquer la stimulation à l'élément ou substrat physique 4 pour qu'il réponde par un signal électrique, celui-ci étant ensuite mesuré par le sous-système d'acquisition 6. En plus d'être de nature différente, c'est-à-dire tension ou courant, le signal de stimulation peut être appliqué sous la forme d'une valeur constante (DC) ou d'une valeur variable dans le temps (AC), comme une onde sinusoïdale, carrée ou pulsée, par exemple. La relation entre les signaux mesurés et le signal de stimulation est la plupart du temps linéaire, ce qui signifie que la réponse mesurée est proportionnelle au signal de signal de stimulation.

Toute variation ou tout bruit apparaissant au sein du système peut masquer les informations pertinentes que le système vise à extraire du signal mesuré, principalement lorsque le niveau du signal mesuré est très faible, inférieur aux microvolts par exemple. En effet, les données acquises par le système sont dépendantes d'une source de tension ou de courant fournie par le système électronique global lui-même. Cette source est conçue pour être la plus précise, la plus stable et la moins bruyante possible, mais elle contient de manière inhérente des variations et du bruit. Ceux-ci sont donc directement injectés dans toutes les variables dépendantes et par conséquent mesurées en retour par le système d'acquisition.

La mesure TIE est basée sur un mécanisme de mesure de l'impédance électrique en plusieurs points au cours duquel la source de stimulation est successivement conduite vers différentes électrodes placées sur le spécimen ou substrat, puis des mesures multiples sont traitées sur la totalité ou une partie des électrodes. Dans la forme de réalisation la plus simple d'un tel système, les signaux passent par des multiplexeurs qui sélectionnent les électrodes à stimuler et celles à mesurer. Enfin, un traitement de données est utilisé pour établir la cartographie de l'impédance du spécimen. Comme mentionné plus haut, ce traitement n'est pas central à l'invention.

L'invention vient résoudre ces problèmes en liant le sous-système de stimulation 2 et le sous-système d'acquisition de données 6 au plus proche du matériau. Ainsi, comme représenté sur la figure 2, un dispositif de mesure de tomographie par processus électrique 10 comprend une source de courant 12, des multiplexeurs 14, 16, 18 et 20, huit électrodes 22 disposées sur un substrat 24 objet de la mesure, et un convertisseur analogique-numérique 26 qui émet un signal numérique de mesure 28.

Dans l'exemple décrit ici, les électrodes 22 sont réparties de manière sensiblement homogène sur le substrat 24. La forme sensiblement circulaire est particulièrement adaptée pour la reconstruction par le système 8. En variante, le dispositif 10 pourrait comprendre moins d'électrodes, par exemple 4, ou plus, par exemple 16 ou plus. De plus, les électrodes pourraient être agencées de manière non homogène et selon d'autres formes que circulairement en fonction des applications.

Les multiplexeurs 14 et 16 sont respectivement reliés en aval et en amont de la source de courant 12, et peuvent être chacun relié à chacune de électrodes 22. Ainsi, chaque paire d'électrodes auquel ils sont reliés définit un circuit de stimulation avec la source de courant 12. De manière similaire, les multiplexeurs 18 et 20 sont respectivement reliés à une entrée « + » et à une entrée « - » du convertisseur analogique-numérique 26. Ainsi, chaque paire d'électrodes auquel ils sont reliés définit un circuit de mesure de la stimulation, cette mesure étant transmise aux bornes « + » et « - » du convertisseur analogique-numérique 26.

Dans l'exemple décrit ici, le convertisseur analogique-numérique 26 fait partie d'un contrôleur qui est agencé pour commander les multiplexeurs 14, 16, 18 et 20. En variante, cette commande pourrait être séparée. Le convertisseur analogique-numérique 26 réalise ainsi la conversion sous forme numérique de la tension mesurée aux bornes des électrodes auxquels sont reliés les multiplexeurs 16 et 18, rapportée à la tension de référence à l'entrée r du convertisseur analogique-numérique 26.

Selon l'invention, cette entrée r est reliée en aval de la source de courant 12, de sorte que celle-ci reçoit sensiblement le même signal que le signal de stimulation réalisé avec les multiplexeurs 14 et 16. Ainsi, le convertisseur analogique-numérique 26 émet en sortie un signal 28 qui est dépourvu de tout bruit affectant la source de courant 12.

Cette approche est totalement nouvelle dans le domaine de la TPE et plus particulièrement de la TIE. Elle est en outre à différencier des équipements employant la mesure ratiométrique conventionnelle. En effet, dans ces équipements, un élément physique de référence forte et absolue est utilisé pour la mesure ratiométrique. Cette référence, différente de l'élément à surveiller, est nécessaire pour détecter des changements minimes dans un système peu affecté par le bruit. La méthodologie et l'enseignement de cette approche sont totalement contraires à ce qui est fait dans l'invention, qui prévoit une stimulation de l'élément à surveiller et l'utilisation de l'effet de la stimulation sur l'élément lui-même comme référence de la mesure ratiométrique. D'autre part, la mesure ratiométrique conventionnelle s'applique à des systèmes dont la connexion à l'élément à surveiller est fixe dans le temps. L'invention, utilisant l'élément à surveiller comme référence, prévoit quant à elle d'élargir le principe de la mesure ratiométrique aux systèmes adaptés à la TPE, au sein desquels la connexion à l'élément à surveiller est faite séquentiellement sur une pluralité d'électrodes.

Pour réaliser les mesures, les multiplexeurs 14, 16, 18 et 20 sont excités de manière séquentielle selon un schéma d'excitation de TIE. Par schéma d'excitation de TIE on entend un schéma choisi parmi :
- un schéma de voisinage selon lequel le courant d'excitation est introduit dans des électrodes voisines, et la chute tension est mesurée successivement dans les autres électrodes, chaque paire d'électrode étant successivement utilisée pour réaliser une excitation,
- un schéma d'opposition selon lequel le courant d'excitation est introduit dans des électrodes diamétralement opposées, et la tension est mesurée successivement dans les autres électrodes, chaque paire d'électrode étant successivement utilisée pour réaliser une excitation, et
- un schéma transverse selon lequel le courant d'excitation est introduit dans des électrodes opposées par rapport à un axe fixé, et la tension est mesurée successivement dans les autres électrodes, chaque paire d'électrode étant successivement utilisée pour réaliser une excitation.

D'autres schémas d'excitation pourront être envisagés.

Dans l'exemple décrit ici, comme la stimulation est réalisée au moyen d'une source de courant continu, la tension de mesure sera mesurée simultanément dans les électrodes. Si cette source de courant est alternative l'amplitude et le décalage de la tension seraient mesurés par rapport au courant alternatif.

La figure 3 représente un deuxième mode de réalisation, qui sera décrit par ses différences. Selon ce mode de réalisation, la source 12 est ici une source de tension, qui peut également être continue ou alternative.

Ce mode de réalisation a l'avantage de fournir une tension directement identique à l'entrée r et aux bornes des multiplexeurs 14 et 16.

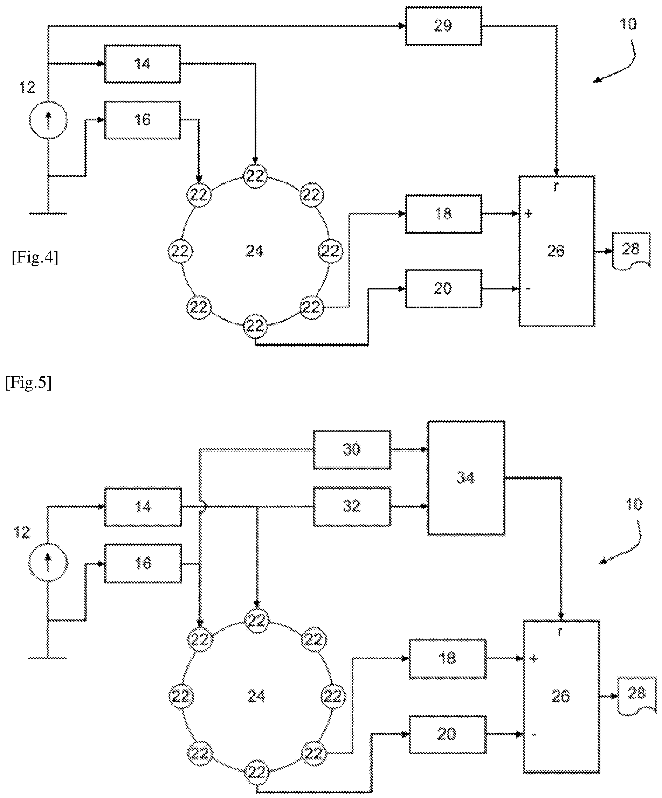

La figure 4 représente un troisième mode de réalisation. Ce mode de réalisation est assez proche du mode de réalisation de la figure 1, mais le dispositif 10 inclut en outre un tampon 29. Le tampon 29 est ici utilisé afin de minimiser l'impact de l'entrée r sur la source de courant 12, afin de garder celle-ci aussi propre et stable que possible.

La figure 5 représente un quatrième mode de réalisation. Dans ce mode de réalisation, afin de transmettre à l'entrée r une tension aussi fidèle que possible que celle correspondant à la stimulation, des multiplexeurs 30 et 32 sont prévus. Ces multiplexeurs sont respectivement reliés en sortie du multiplexeur 14 et du multiplexeur 16, et sont reliés aux entrées d'un amplificateur opérationnel 34 qui garantit ainsi que la tension soumise à l'entrée r est bien directement proportionnelle à la tension de stimulation à laquelle le substrat 24 est soumis. Les multiplexeurs 30 et 32 sont excités en correspondance avec les multiplexeurs 16 et 14 respectivement. Ce mode de réalisation a l'avantage de limiter l'influence des chutes de tension qui peuvent se produire sur le multiplexeur 14 et le multiplexeur 16 du fait de l'envoi d'un courant fort vers le substrat 24.

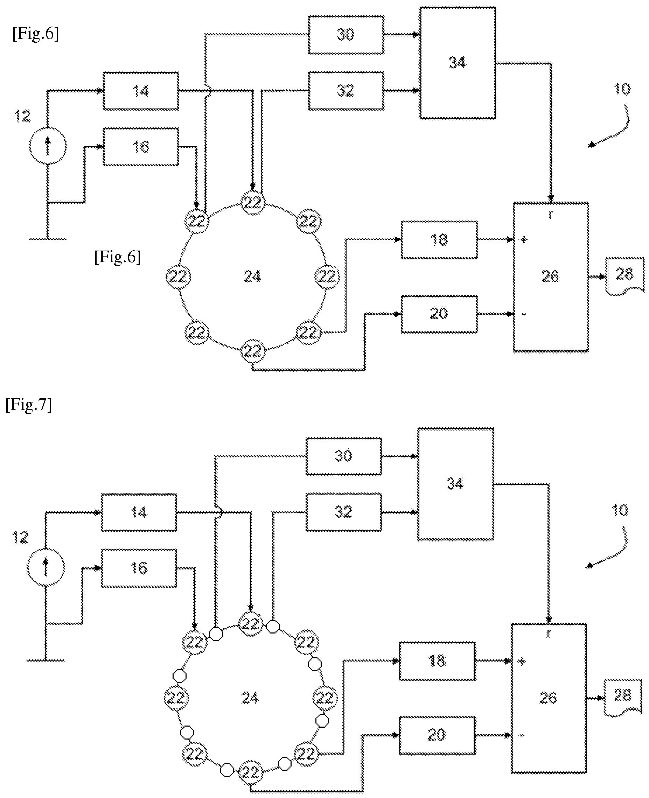

La figure 6 représente un cinquième mode de réalisation. Dans ce mode de réalisation, les multiplexeurs 30 et 32 sont directement reliés aux électrodes 22 auxquelles sont reliés les multiplexeurs 16 et 14. Ce mode de réalisation a l'avantage de fournir à l'entrée r une tension intégrant non seulement le bruit généré par les multiplexeurs 14 et 16 mais aussi celui dû aux interactions entre les multiplexeurs 14 et 16 et le substrat 24. Les variations supplémentaires que sont le bruit et les chutes de tension induits par les multiplexeurs 14 16 situés entre la source de courant 12 et le substrat 24 sont ainsi injectées dans la tension de référence du convertisseur analogique-numérique 26.

Enfin, la figure 7 représente un sixième mode de réalisation. Dans ce mode de réalisation, les électrodes 22 du substrat 24 sont chacune dupliquées en disposant une série supplémentaire d'électrodes selon la même configuration que les électrodes 22, mais en léger décalage par rapport aux électrodes 22. Chaque électrode 22 se voit ainsi associer une électrode de la série supplémentaires d'électrodes. Dans ce mode de réalisation, les multiplexeurs 30 et 32 sont reliés aux électrodes associées aux électrodes 22 auxquelles sont reliés les multiplexeurs 16 et 14. Ce mode de réalisation a l'avantage de fournir à l'entrée r une tension réelle vue du substrat 24. Ce mode de réalisation écarte, de par sa nature, le bruit généré par le passage du signal de stimulation au travers des multiplexeurs 14 et 16, entre les multiplexeurs 14 et 16 et le substrat 24 et enfin au travers des résistances de contact entre les électrodes 22 et le substrat 24.

Dans ce qui précède, il doit être compris que le substrat 24 peut être une interface homme-machine ou un substrat sur lequel on cherche à faire du CND ou un substrat dont on cherche à surveiller la santé structurelle. Ainsi, bien que les exemples illustrés concernent une application de type TIE, le dispositif 10 trouve à s'appliquer :
- à toute interface homme-machine IHM visant à stimuler l'interface physique et à mesurer en retour les signaux corrélés résultant de la stimulation de l'interface physique afin de mesurer toute variation d'impédance locale à l'intérieur de l'interface, et comprenant une source de stimulation électrique continue ou variable et un convertisseur analogique-numérique,
- à toute IHM basée sur la tomographie d'impédance électrique et visant à stimuler l'interface physique et à mesurer les signaux corrélés résultant de la stimulation de l'interface physique afin de mesurer toute variation d'impédance locale au sein de l'interface, et comprenant une source de stimulation électrique continue ou variable et un convertisseur analogique-numérique,

- à toute IHM basée sur la tomographie par résistance électrique et visant à stimuler l'interface physique et à mesurer les signaux corrélés en retour résultant de la stimulation de l'interface physique afin de mesurer toute variation d'impédance locale dans l'interface, et comprenant une source de stimulation électrique continue ou variable et un convertisseur analogique-numérique,
- à toute IHM basée sur la tomographie à capacité électrique et visant à stimuler l'interface physique et à mesurer les signaux corrélés en retour résultant de la stimulation de l'interface physique afin de mesurer toute variation d'impédance locale au sein de l'interface, et comprenant une source de stimulation électrique variable et un convertisseur analogique-numérique,
- à tout système de surveillance de la santé structurelle (SHM) visant à stimuler un spécimen physique et de mesurer en retour les signaux corrélés résultant de la stimulation du composant physique afin de mesurer toute variation d'impédance locale à l'intérieur de l'échantillon, et comprenant une source de stimulation électrique continue ou variable et un convertisseur analogique-numérique,
- à tout système SHM basé sur la tomographie d'impédance électrique et visant à stimuler un spécimen physique et à mesurer en retour les signaux corrélés résultant de la stimulation de la composante physique afin de mesurer toute variation d'impédance locale à l'intérieur du spécimen, et comprenant une source de stimulation électrique continue ou variable et un convertisseur analogique-numérique,
- à tout système SHM basé sur la tomographie par résistance électrique et visant à stimuler un spécimen physique et à mesurer en retour les signaux corrélés résultant de la stimulation de l'élément physique afin de mesurer toute variation d'impédance locale à l'intérieur du spécimen, et comprenant une source de stimulation électrique continue ou variable et un convertisseur analogique-numérique,
- à tout système SHM basé sur la tomographie par capacité électrique et visant à stimulant un spécimen physique et à mesurer en retour les signaux corrélés résultant de la stimulation de l'élément physique afin de mesurer toute variation d'impédance locale à l'intérieur du spécimen, et comprenant une source de stimulation électrique variable et un convertisseur analogique-numérique,
- à tout système de contrôle non destructif (CND) visant à stimuler un spécimen physique et de mesurer en retour les signaux corrélés résultant de la stimulation du composant physique afin de mesurer toute variation d'impédance locale à l'intérieur de l'échantillon, et comprenant une source de stimulation électrique continue ou variable et un convertisseur analogique-numérique,
- à tout système CND basé sur la tomographie d'impédance électrique et visant à stimuler un spécimen physique et à mesurer en retour les signaux corrélés résultant de la stimulation de la composante physique afin de mesurer toute variation d'impédance locale à l'intérieur du spécimen, et comprenant une source de stimulation électrique continue ou variable et un convertisseur analogique-numérique,
- à tout système CND basé sur la tomographie par résistance électrique et visant à stimuler un spécimen physique et à mesurer en retour les signaux corrélés résultant de la stimulation de l'élément physique afin de mesurer toute variation d'impédance locale à l'intérieur du spécimen, et comprenant une source de stimulation électrique continue ou variable et un convertisseur analogique-numérique, et
- à tout système CND basé sur la tomographie par capacité électrique et visant à stimulant un spécimen physique et à mesurer en retour les signaux corrélés résultant de la stimulation de l'élément physique afin de mesurer toute variation d'impédance locale à l'intérieur du spécimen, et comprenant une source de stimulation électrique variable et un convertisseur analogique-numérique,

## Revendications

1. Dispositif de mesure de tomographie par processus électrique pour substrat solide, comprenant une source de courant ou de tension (12), une pluralité d'électrodes (22) propres à être reliées à un substrat solide à mesurer (24) et un convertisseur analogique numérique (26) comprenant deux entrées de mesure (+,-) et une entrée de référence (r), agencé pour émettre un signal numérique correspondant à la différence entre la tension des deux entrées de mesure (+,-) proportionnellement à un niveau de tension désigné par l'entrée de référence (r), la source de courant ou de tension (12) pouvant être reliée à une paire d'électrodes dans la pluralité d'électrodes (22) de manière commandée, de sorte qu'un courant issu de la source de courant ou de tension (12) traverse ledit substrat à mesurer selon une séquence d'excitation choisie, les deux entrées de mesure (+,-) du convertisseur analogique numérique (26) pouvant chacune être reliée à une électrode respective d'une paire d'électrodes dans la pluralité d'électrodes (22) choisie en fonction de ladite séquence d'excitation, **caractérisé en ce que** la source de courant ou de tension (12) est reliée à l'entrée de référence (r) du convertisseur analogique numérique (26),
le dispositif comprenant en outre un premier multiplexeur (14) connecté en aval de la source de courant ou de tension (12) et relié à la pluralité d'électrodes (22) et un deuxième multiplexeur (16) connecté en amont de la source de courant ou de tension (12) et relié à la pluralité d'électrodes (22) afin de réaliser la séquence d'excitation, le dispositif est **caractérisé en ce qu'**il comprend en outre un amplificateur opérationnel (34) dont la sortie est reliée à l'entrée de référence (r) du convertisseur analogique numérique (26) et dont les entrées sont reliées à une électrode respective de la paire d'électrodes dans la pluralité d'électrodes (22) selon ladite séquence d'excitation, et
en outre un troisième multiplexeur (30) connecté entre la sortie du premier multiplexeur (14) et l'une des entrées de l'amplificateur opérationnel (34), et un quatrième multiplexeur (32) connecté entre la sortie du deuxième multiplexeur (16) et l'autre entrée de l'amplificateur opérationnel (34).

2. Dispositif selon la revendication 1, comprenant en outre un cinquième multiplexeur (18) connecté à l'une des entrées de mesure (+) du convertisseur analogique numérique (26) et relié à la pluralité d'électrodes (22) et un sixième multiplexeur (20) connecté à l'autre des entrées de mesure (-) du convertisseur analogique numérique (26) et relié à la pluralité d'électrodes (22), permettant de réaliser la liaison choisie en fonction de ladite séquence d'excitation.

3. Dispositif selon la revendication 1 ou 2, comprenant en outre un tampon (29) disposé entre la source de courant ou de tension (12) et l'entrée de référence (r) du convertisseur analogique numérique (26).

4. Dispositif selon l'une des revendications précédentes, dans lequel la source de courant ou de tension (12) est continue.

5. Dispositif selon l'une des revendications 1 à 3, dans lequel la source de courant ou de tension (12) est alternative.

6. Dispositif selon l'une des revendications précédentes, dans lequel le dispositif de mesure (10) met en œuvre une mesure d'interface homme-machine.

7. Dispositif selon l'une des revendications 1 à 5, dans lequel le dispositif de mesure (10) met en œuvre une mesure de surveillance de santé structurelle ou de contrôle non destructif.

## Patentansprüche

1. Elektrische Prozesstomographie-Messvorrichtung für Festsubstrate, umfassend eine Strom- oder Spannungsquelle (12), eine Vielzahl von Elektroden (22), die mit einem zu messenden Festsubstrat (24) verbunden werden können, und einen Analog-Digital-Wandler (26) umfassend zwei Messeingänge (+,-) und einen Referenzeingang (r), der so angeordnet ist, dass er ein digitales Signal ausgibt, das der Differenz zwischen der Spannung der beiden Messeingänge (+,-) proportional zu einem durch den Referenzeingang (r) bezeichneten Spannungspegel entspricht, wobei die Strom- oder Spannungsquelle (12) mit einem Elektrodenpaar aus der Vielzahl von Elektroden (22) steuerbar verbindbar ist, so dass ein von der Strom- oder Spannungsquelle (12) stammender Strom das zu messende Substrat gemäß einer ausgewählten Anregungssequenz durchläuft, wobei die beiden Messeingänge (+,-) des Analog-Digital-Wandlers (26) jeweils mit einer jeweiligen Elektrode eines Elektrodenpaars aus der Vielzahl von Elektroden (22) verbunden werden können, das in Abhängigkeit von der Anregungssequenz ausgewählt ist, **dadurch gekennzeichnet, dass** die Strom- oder Spannungsquelle (12) mit dem Referenzeingang (r) des Analog-Digital-Wandlers (26) verbunden ist,
die Vorrichtung ferner umfassend einen ersten Multiplexer (14), der der Strom- oder Spannungsquelle (12) nachgeschaltet und mit der Vielzahl von Elektroden (22) verbunden ist, und einen zweiten Multiplexer (16), der der Strom- oder Spannungsquelle (12) vorgeschaltet und mit der Vielzahl von Elektroden (22) verbunden ist, um die Anregersequenz durchzuführen,
die Vorrichtung **dadurch gekennzeichnet ist, dass** sie ferner einen betriebsbereiten Verstärker (34) umfasst, dessen Ausgang mit dem Referenzeingang (r) des Analog-Digital-Wandlers (26) verbunden ist und dessen Eingänge mit einer jeweiligen Elektrode des Elektrodenpaars aus der Vielzahl von Elektroden (22) gemäß der Anregungssequenz verbunden sind, und ferner einen dritten Multiplexer (30), der zwischen dem Ausgang des ersten Multiplexers (14) und einem der Eingänge des betriebsbereiten Verstärkers (34) verbunden ist, und einen vierten Multiplexer (32), der zwischen dem Ausgang des zweiten Multiplexers (16) und dem anderen Eingang des betriebsbereiten Verstärkers (34) verbunden ist.

2. Vorrichtung nach Anspruch 1, ferner umfassend einen fünften Multiplexer (18), der mit einem der Messeingänge (+) des Analog-Digital-Wandlers (26) verbunden und mit der Vielzahl von Elektroden (22) verbunden ist, und einen sechsten Multiplexer (20), der mit dem anderen der Messeingänge (-) des Analog-Digital-Wandlers (26) verbunden und mit der Vielzahl von Elektroden (22) verbunden ist, wodurch die entsprechend der Anregungssequenz ausgewählte Verbindung hergestellt werden kann.

3. Vorrichtung nach Anspruch 1 oder 2, ferner umfassend einen Puffer (29), der zwischen der Strom- oder Spannungsquelle (12) und dem Referenzeingang (r) des Analog-Digital-Wandlers (26) angeordnet ist.

4. Vorrichtung nach einem der vorstehenden Ansprüche, wobei die Strom- oder Spannungsquelle (12) gleichgerichtet ist.

5. Vorrichtung nach einem der Ansprüche 1 bis 3, wobei die Strom- oder Spannungsquelle (12) wechselgerichtet ist.

6. Vorrichtung nach einem der vorhergehenden Ansprüche, wobei die Messvorrichtung (10) eine Mensch-Maschine-Schnittstellenmessung umsetzt.

7. Vorrichtung nach einem der Ansprüche 1 bis 5, wobei die Messvorrichtung (10) eine strukturelle Gesundheitsüberwachungs- oder zerstörungsfreie Kontrollmaßnahme umsetzt.

## Claims

1. An electrical process tomography measurement device for a solid substrate, comprising a current or voltage source (12), a plurality of electrodes (22) suitable for connection to a solid substrate to be measured (24), and an analogue-to-digital converter (26) comprising two measurement inputs (+,-) and a reference input (r) and arranged to emit a digital signal corresponding to the difference between the voltage of the two measurement inputs (+,-) proportionally to a voltage level designated by the reference input (r), wherein the current or voltage source (12) can be connected to a pair of electrodes in the plurality of electrodes (22) in a controlled manner, so that a current from the current or voltage source (12) passes through said substrate to be measured according to a selected excitation sequence, wherein the two measurement inputs (+,-) of the analogue-to-digital converter (26) can each be connected to a respective electrode of a pair of electrodes in the plurality of electrodes (22) selected based on said excitation sequence, **characterised in that** the current or voltage source (12) is connected to the reference input (r) of the analogue-to-digital converter (26),
the device further comprising a first multiplexer (14) connected downstream of the current or voltage source (12) and linked to the plurality of electrodes (22) and a second multiplexer (16) connected upstream of the current or voltage source (12) and linked to the plurality of electrodes (22) in order to perform the excitation sequence,
the device is **characterised in that** it further comprises an operational amplifier (34) whose output is connected to the reference input (r) of the analogue-to-digital converter (26) and whose inputs are connected to a respective electrode of the pair of electrodes in the plurality of electrodes (22) according to said excitation sequence, and further a third multiplexer (30) connected between the output of the first multiplexer (14) and one of the inputs of the operational amplifier (34), and a fourth multiplexer (32) connected between the output of the second multiplexer (16) and the other input of the operational amplifier (34).

2. The device according to claim 1, further comprising a fifth multiplexer (18) connected to one of the measurement inputs (+) of the analogue-to-digital converter (26) and connected to the plurality of electrodes (22) and a sixth multiplexer (20) connected to the other of the measurement inputs (-) of the analogue-to-digital converter (26) and linked to the plurality of electrodes (22), making it possible to make the connection selected based on said excitation sequence.

3. The device according to claim 1 or 2, further comprising a buffer (29) positioned between the current or voltage source (12) and the reference input (r) of the analogue-to-digital converter (26).

4. The device according to one of the preceding claims, wherein the current or voltage source (12) is a DC source.

5. The device according to one of claims 1 to 3, in which the current or voltage source (12) is an AC source.

6. The device according to one of the preceding claims, wherein the measuring device (10) implements a human-machine interface measurement.

7. The device according to one of claims 1 to 5, wherein the measuring device (10) implements structural or non-destructive testing health monitoring measurement.
